# EUROPEAN PATENT APPLICATION

(11) **EP 4 431 113 A1**
(43) Date of publication of application: **18.09.2024**
(21) Application number: 21963512.5
(22) Date of filing: 09.11.2021
(51) Int. Cl.: A61K 45/06, A61K 31/427, A61K 31/4375, A61K 31/55, A61K 31/551, A61K 31/58, A61K 31/69, A61K 38/05, A61P 31/04

(54) **PHARMACEUTICAL COMPOSITION CONTAINING ß-LACTAM COMPOUND AND USE THEREOF**

(71) Applicant: Institute of Medicinal Biotechnology, Chinese Academy of Medical Sciences, Beijing 100050 (CN); Guangzhou HC New Drug Research Co., Ltd., Guangzhou, Guangdong 510700 (CN)
(72) Inventor: SONG, Danqing, Beijing 100050 (CN); JIANG, Jiandong, Beijing 100050 (CN); WANG, Yanxiang, Beijing 100050 (CN); YOU, Xuefu, Beijing 100050 (CN); FENG, Shengxi, Guangzhou, Guangdong 510520 (CN); LI, Yinghong, Beijing 100050 (CN); LU, Xi, Beijing 100050 (CN); LIN, Lidong, Guangzhou, Guangdong 510520 (CN); FAN, Tianyun, Beijing 100050 (CN); PANG, Jing, Beijing 100050 (CN); GUO, Zhihao, Beijing 100050 (CN); LI, Zhiwen, Beijing 100050 (CN); LIU, Yonghua, Beijing 100050 (CN); WANG, Xiukun, Beijing 100050 (CN)
(74) Representative: Nederlandsch Octrooibureau
(86) International application number: PCT/CN2021/129609
(87) International publication number: WO 2023/082055

(57) **Abstract**

The present invention relates to a pharmaceutical composition comprising a β-lactam compound of formula (I), or a stereoisomer, a solvate or a pharmaceutically acceptable salt or ester thereof, and a lactamase inhibitor or an efflux pump inhibitor, and the use thereof for inhibiting microorganisms, particularly bacteria, especially Gram-negative bacteria, wherein each symbol in formula (I) is as defined in the description.

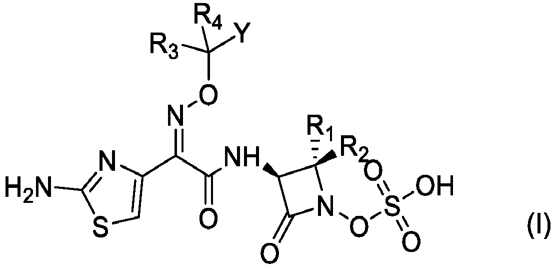

## Description

### Technical Field

The present invention relates to a pharmaceutical composition comprising a β-lactam compound and the use thereof for inhibiting microorganisms, particularly bacteria, especially Gram-negative bacteria.

### Background Art

β-lactam antibiotics (β-lactams) refer to a large class of antibiotics that contain a β-lactam ring in their chemical structure. Many β-lactam antibiotics are known. Such antibiotics have the advantages of strong bactericidal activities, low toxicity, use for a wide range of indications and good clinical efficacy.

Nevertheless, with the widespread clinical use of the antibiotics, there has emerged some drug resistance, and the antimicrobial effects in some aspects are not always satisfactory. Therefore, there is a need to develop new antimicrobial drugs with excellent antimicrobial effects.

### Summary of the Invention

The present invention is made in order to overcome the aforesaid deficiencies in the prior art.

The present invention provides a pharmaceutical composition comprising
(a) a compound of formula (I), or a stereoisomer, a solvate or a pharmaceutically acceptable salt or ester thereof, 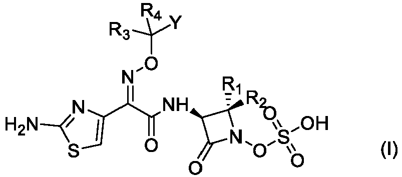 wherein
   R₁ and R₂ each independently represent hydrogen, alkyl, aryl, alkoxy, aryloxy, arylalkyl, alkylcarbonylalkyl, alkylacyloxyalkyl or alkoxyacyloxyalkyl, heterocyclyl or heteroaryl, or R₁ and R₂ together form cycloalkyl, wherein all the aforementioned groups are optionally substituted;
   R₃ and R₄ each independently represent hydrogen, alkyl, aryl, alkoxy, aryloxy, arylalkyl, alkylcarbonylalkyl, alkylacyloxyalkyl or alkoxyacyloxyalkyl, heterocyclyl or heteroaryl, or R₃ and R₄ together form cycloalkyl, wherein all the aforementioned groups are optionally substituted; and
   Y represents optionally substituted alkenyl or alkynyl, or represents carboxyl or ester; and
(b) a lactamase inhibitor or an efflux pump inhibitor.

The pharmaceutical composition of the present invention can be used as is or together with a pharmaceutically acceptable carrier, an excipient or other adjuvants.

The present invention also provides the use of the pharmaceutical composition of the present invention as an antimicrobial agent.

In addition, the present invention also provides the use of the pharmaceutical composition of the present invention in the manufacture of a medicament for treating infectious diseases.

Furthermore, the present invention also provides a method for treating infectious diseases, comprising administering the pharmaceutical composition of the present invention to humans or animals.

It has been surprisingly found that the compound of formula (I), or a stereoisomer, a solvate, or a pharmaceutically acceptable salt or ester thereof, and the lactamase inhibitor or efflux pump inhibitor in the pharmaceutical composition of the present invention exhibit a synergistic antimicrobial effect.

### Detailed Description of the Invention

### General definitions

The compound of formula (I) of the present application, and a stereoisomer, a solvate and a pharmaceutically acceptable salt or an ester thereof are sometimes collectively referred to as the "compound of the present invention".

The "pharmaceutical composition of the present invention" comprises the compound of the present invention and a lactamase inhibitor or an efflux pump inhibitor, and optionally a pharmaceutically acceptable carrier, an excipient and/or other adjuvants.

As used herein, the term "optional" or "optionally" means that the subsequently described event, circumstance, or substance may or may not occur or be present, and that the description includes instances where the event, circumstance, or substance occurs or is present and instances where it does not occur or is not present.

As used herein, the term "comprise" and its synonyms "comprising" and "comprises", mean "including but not limited to," and are not intended to exclude, for example, other additives, components, integers or steps.

In the present invention, the "microorganism" has a well-known meaning in the art, including bacteria, viruses and fungi, especially bacteria, such as Gram-positive bacteria, Gram-negative bacteria, and the like

In the context of the present invention, unless specified otherwise, the term "alkyl", either on its own or in combination with further terms, for example arylalkyl, is understood to mean a radical of a saturated, aliphatic hydrocarbon group which may be branched or unbranched. Examples of (C₁-C₁₂)-alkyl radicals are methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl, hexyl, n-heptyl, n-octyl, n-nonyl, n-decyl, n-undecyl and n-dodecyl. Among these alkyl radicals, (C₁-C₆)-alkyl radicals are particularly preferred. Especially preferred are (C₁-C₄)-alkyl radicals.

In the context of the present invention, unless specified otherwise, the term "cycloalkyl", either on its own or in combination with further terms, for example arylcycloalkyl, is understood to mean a radical of a saturated aliphatic cyclic hydrocarbon group which has from 3 to 8 carbocyclic atoms and may optionally be substituted. Examples of (C₃-C₈)-cycloalkyl radicals are cyclopropyl, cyclobutyl, cyclopentyl, cyclohexyl, C₇-cycloalkyl and Cs-cycloalkyl. These radicals may optionally be substituted.

In the present invention, the term "alkoxy", either on its own or in combination with further terms, for example carboxyalkoxyimino, is understood to mean alkyl-O-, where the term "alkyl" is as defined above. In particular, the alkyl here may be linear or branched.

According to the present invention, unless specified otherwise, "alkylcarbonyl" (alkyl-C(=O)-) represents alkyl radicals bonded to the skeleton via -C(=O)-, such as (C₁-C₁₀)-, (C₁-C₆)- or (C₁-C₄)-alkylcarbonyl. The number of the carbon atoms here relates to the alkyl radical in the alkylcarbonyl group.

According to the present invention, the term "alkylacyloxy" (alkyl-C(=O)-O-), unless specified otherwise, represents alkyl radicals bonded to the skeleton via the oxygen of an acyloxy group (-C(=O)-O-), such as (C₁-C₁₀)-, (C₁-C₆)- or (C₁-C₄)-alkylacyloxy. The number of the carbon atoms here relates to the alkyl radical in the alkylacyloxy group.

According to the present invention, unless specified otherwise, the term "alkoxyacyloxy" (alkoxy-C(=O)-O-) represents alkoxy radicals bonded to the skeleton via the oxygen of an acyloxy group (-C(=O)-O-), such as (C₁-C₁₀)-, (C₁-C₆)- or (C₁-C₄)- alkoxyacyloxy. Here, the number of the carbon atoms relates to the alkyl radical in the alkoxyacyloxy group.

According to the present invention, unless specified otherwise, the term "alkenyl" is understood to mean hydrocarbon radicals having at least one carbon-carbon double bond. Preferably, examples of alkenyl are vinyl, propenyl, isopropenyl, allyl, n-butenyl, isobutenyl, 2-methylpropenyl, and the like.

According to the present invention, unless specified otherwise, the term "alkynyl" is understood to mean hydrocarbon radicals having at least one carbon-carbon triple bond. Preferably, examples of alkynyl are ethynyl, propynyl, isopropynyl, propargyl, n-butynyl, isobutynyl, 2-methylpropynyl and the like.

According to the present invention, unless specified otherwise, the term "ester" is understood to mean a group having the structure -COOR₅, wherein R₅ is alkyl as defined above. R₅ is preferably (C₁-C₁₂)-alkyl, examples of which are methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl, hexyl, n-heptyl, n-octyl, n-nonyl, n-decyl, n-undecyl and n-dodecyl. More preferably, R₅ is (C₁-C₆)-alkyl. Particularly preferably, R₅ is (C₁-C₆)-alkyl.

Unless specified otherwise, "heterocyclyl" means a saturated or partially saturated monocyclic ring containing carbon atoms and at least one heteroatom in the ring. Preferably, the heterocyclyl contains 3, 4, 5, 6 or 7 carbon atoms and 1 or 2 heteroatoms selected from oxygen, sulfur and nitrogen. Examples of heterocyclyl are azetidinyl, azacyclopentyl, azacyclohexyl, oxetanyl, oxolanyl, oxacyclohexyl, dioxacyclohexyl, thietanyl, thiacyclopentyl, thiacyclohexyl and tetrahydrofuranyl.

According to the present invention, unless specified otherwise, the term "aryl" is understood to mean an aromatic radical having 6 to 14 carbon atoms, preferably phenyl, naphthyl, anthryl or phenanthryl, more preferably phenyl.

Unless specified otherwise, the term "arylalkyl" is understood to mean a combination of "aryl" and "alkyl" radicals as defined in accordance with the present invention, where the radical is generally bonded via the alkyl group. Examples thereof are benzyl, phenylethyl or α-methylbenzyl, particular preference being given to benzyl.

Unless specified otherwise, "heteroaryl" means a monocyclic, bicyclic or tricyclic heterocyclic group containing carbon atoms and at least one heteroatom, wherein at least one ring is aromatic. Preferably, heteroaryl contains 3, 4, 5 or 6 carbon atoms and is selected from furyl, thienyl, pyrrolyl, pyrazolyl, imidazolyl, 1,2,3-triazolyl, 1,2,4-triazolyl, oxazolyl, isoxazolyl, thiazolyl, isothiazolyl, 1,2,3-oxadiazolyl, 1,2,4-oxadiazolyl, 1,3,4-oxadiazolyl, 1,2,5-oxadiazolyl, 1,2,3-thiadiazolyl, 1,2,4-thiadiazolyl, 1,3,4-thiadiazolyl, 1,2,5-thiadiazolyl, pyridyl, pyrimidinyl, pyridazinyl, pyrazinyl, 1,2,3-triazinyl, 1,2,4-triazinyl, 1,3,5-triazinyl, tetrazolyl, benzofuryl, benzisofuryl, benzothienyl, benzisothienyl, indolyl, isoindolyl, indazolyl, benzothiazolyl, benzisothiazolyl, benzoxazolyl, benzisoxazolyl, benzimidazolyl, 2,1,3-benzoxadiazole, quinolinyl, isoquinolyl, cinnolinyl, phthalazinyl, quinazolinyl, quinoxalinyl, naphthyridinyl, benzotriazinyl, purinyl, pteridinyl and indolizinyl.

When a base structure is "substituted", this in each case includes simultaneous substitution by one or more identical and/or structurally different radicals.

The term "solvate" refers to forms of the compound, or a salt thereof that are associated with a solvent, usually generated by a solvolysis reaction. This physical association may include hydrogen bonding. Conventional solvents include water, methanol, ethanol, acetic acid, DMSO, THF, diethyl ether, and the like. The compounds described herein may be prepared, e.g., in crystalline form, and may be solvated. Suitable solvates include pharmaceutically acceptable solvates and further include both stoichiometric solvates and non-stoichiometric solvates. In certain instances, the solvate will be capable of isolation, for example, when one or more solvent molecules are incorporated in the crystal lattice of a crystalline solid. "Solvate" encompasses both solvates in solution and isolatable solvates. Representative solvates include hydrates, ethanolates, and methanolates.

If the compound can form, through a hydrogen shift, tautomers whose structure is not formally covered by the formula (I), these tautomers are still covered by the definition of the compound of the formula (I) of the present invention, unless a particular tautomer is considered otherwise. For example, many carbonyl compounds may be present both in the keto form and in the enol form, both forms being encompassed by the definition of the compound of the formula (I).

Depending on the nature of the substituents and the manner in which they are attached, the compound of the formula (I) may be present as stereoisomers. The possible stereoisomers defined by the specific three-dimensional form thereof, such as enantiomers, diastereomers, Z and E isomers, are all encompassed by the formula (I). If, for example, one or more alkenyl groups are present, diastereomers (Z and E isomers) may occur. If, for example, one or more asymmetric carbon atoms are present, enantiomers and diastereomers may occur. Stereoisomers can be obtained from the mixtures obtained in the preparation by customary separation methods. The chromatographic separation can be effected either on the analytical scale to find the enantiomeric excess or the diastereomeric excess, or else on the preparative scale to produce test specimens for biological testing. It is likewise possible to selectively prepare stereoisomers by using stereoselective reactions with use of optically active starting materials and/or adjuvants. The present invention thus also relates to all stereoisomers which are embraced by the general formula (I) but are not shown in their specific stereomeric form, and to mixtures thereof.

If appropriate, the compound of the formula (I) may be present in various polymorphic forms or as a mixture of various polymorphic forms. Both the pure polymorphs and the polymorph mixtures are provided by the present invention and can be used in accordance with the present invention.

In the context of the present invention, the term "synergy" has the meaning generally understood by those skilled in the art, and for example, means the interaction of two components that when combined produce a total effect that is greater than the sum of the effects of the individual components.

In the present invention, unless otherwise stated, all operations were carried out at room temperature and under normal pressure.

In the present invention, unless otherwise stated, the ratios of each component in the compositions and mixtures are by weight.

### Pharmaceutical composition

In one aspect, the present invention provides a pharmaceutical composition comprising
(a) a compound of formula (I), or a stereoisomer, a solvate or a pharmaceutically acceptable salt or ester thereof, 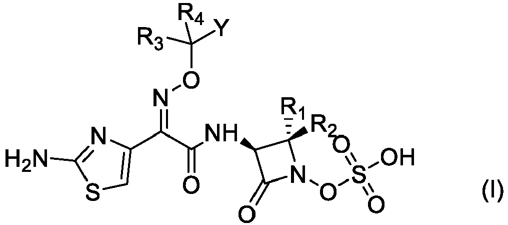 wherein
   R₁ and R₂ each independently represent hydrogen, alkyl, aryl, alkoxy, aryloxy, arylalkyl, alkylcarbonylalkyl, alkylacyloxyalkyl or alkoxyacyloxyalkyl, heterocyclyl or heteroaryl, or R₁ and R₂ together form cycloalkyl, wherein all the aforementioned groups are optionally substituted;
   R₃ and R₄ each independently represent hydrogen, alkyl, aryl, alkoxy, aryloxy, arylalkyl, alkylcarbonylalkyl, alkylacyloxyalkyl or alkoxyacyloxyalkyl, heterocyclyl or heteroaryl, or R₃ and R₄ together form cycloalkyl, wherein all the aforementioned groups are optionally substituted;
   Y represents optionally substituted alkenyl or alkynyl, or represents carboxyl or ester; and
(b) a lactamase inhibitor or an efflux pump inhibitor.

### Component (a)

The pharmaceutical composition of the present invention comprises component (a): a compound of formula (I), or a stereoisomer, a solvate or a pharmaceutically acceptable salt or ester thereof. The compound of formula (I) described above provides a general definition of the compound of the present invention. Preferred, more preferred, particularly preferred and most preferred substituents or ranges of the radicals listed in the formula (I) of the present invention are illustrated below.

Preferably, R₁ and R₂ each independently represent hydrogen or the following optionally substituted groups: (C₁-C₁₂)-alkyl, (C₆-C₁₄)-aryl, (C₁-C₁₂)-alkoxy, (C₁-C₁₂)-alkylcarbonyl-(C₁-C₁₂)-alkyl, (C₁-C₁₂)-alkylacyloxy-(C₁-C₁₂)-alkyl or (C₁-C₁₂)-alkoxyacyloxy-(C₁-C₁₂)-alkyl, or R₁ and R₂ together form (C₃-C₈) cycloalkyl.

More preferably, R₁ and R₂ each independently represent hydrogen, or optionally substituted (C₁-C₁₀)-alkyl, such as optionally substituted methyl, ethyl, n-propyl, isopropyl, n-butyl, iso-butyl, sec-butyl, tert-butyl, n-pentyl, isopentyl, neopentyl, tert-pentyl, 1-methylbutyl, 2-methylbutyl, 1-ethylpropyl, 1,2-dimethylpropyl, hexyl, n-heptyl, n-octyl, n-nonyl or n-decyl; or R₁ and R₂ together form (C₃-C₆)cycloalkyl.

Particularly preferably, R₁ and R₂ each independently represent hydrogen, or optionally substituted methyl, ethyl, n-propyl, isopropyl, n-butyl, iso-butyl, sec-butyl or tert-butyl; or R₁ and R₂ together form (C₃-C₆)cycloalkyl.

Most preferably, R₁ and R₂ each independently represent hydrogen, or optionally substituted methyl or ethyl.

Preferably, R₃ and R₄ each independently represent hydrogen or the following optionally substituted groups: (C₁-C₁₂)-alkyl, (C₆-C₁₄)-aryl, (C₁-C₁₂)-alkoxy, (C₁-C₁₂)-alkylcarbonyl-(C₁-C₁₂)-alkyl, (C₁-C₁₂)-alkylacyloxy-(C₁-C₁₂)-alkyl or (C₁-C₁₂)-alkoxyacyloxy-(C₁-C₁₂)-alkyl, or R₃ and R₄ together form (C₃-C₈)cycloalkyl.

More preferably, R₃ and R₄ each independently represent hydrogen, optionally substituted (C₁-C₁₀)-alkyl, such as optionally substituted methyl, ethyl, n-propyl, isopropyl, n-butyl, iso-butyl, sec-butyl, tert-butyl, n-pentyl, isopentyl, neopentyl, tert-pentyl, 1-methylbutyl, 2-methylbutyl, 1-ethylpropyl, 1,2-dimethylpropyl, hexyl, n-heptyl, n-octyl, n-nonyl or n-dec; or optionally substituted (C₆-C₁₂)-aryl; or R₃ and R₄ together form (C₃-C₆)cycloalkyl.

Particularly preferably, R₃ and R₄ each independently represent hydrogen, optionally substituted methyl, ethyl, n-propyl, isopropyl, n-butyl, iso-butyl, sec-butyl or tert-butyl; or optionally substituted (C₆-C₁₂)-aryl; or R₃ and R₄ together form (C₃-C₆)cycloalkyl.

Most preferably, R₃ and R₄ each independently represent hydrogen, or optionally substituted methyl, ethyl, n-propyl or isopropyl; or R₃ and R₄ together form (C₃-C₆)cycloalkyl.

Preferably, Y represents optionally substituted alkenyl or alkynyl, or represents carboxyl or ester.

More preferably, Y represents vinyl, propenyl, isopropenyl, allyl, n-butenyl, isobutenyl, 2-methylpropenyl, ethynyl, propynyl, isopropynyl, propargyl, n-butynyl, isobutynyl, 2-methylpropynyl, carboxyl, or -COOR₅, wherein R₅ is (C₁-C₁₂)-alkyl, examples of which are methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl, hexyl, n-heptyl, n-octyl, n-nonyl, n-decyl, n-undecyl and n-dodecyl.

Particularly preferably, Y represents vinyl, propenyl, isopropenyl, allyl, n-butenyl, isobutenyl, 2-methylpropenyl, ethynyl, propynyl, isopropynyl, propargyl, n-butynyl, isobutynyl, 2-methylpropynyl, carboxyl, or -COOR₅, wherein R₅ is (C₁-C₁₂)-alkyl, examples of which are methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl and hexyl.

Most preferably, Y represents vinyl, propenyl, ethynyl, propynyl, carboxyl or -COOR₅, wherein R₅ is methyl, ethyl, n-propyl or isopropyl.

The abovementioned respective general definitions or various preferred definitions or illustrations of radicals can be combined with each other as desired; that is, combinations between the respective general ranges or various preferred ranges can be included. They apply both to the compounds of formula (I) and to the corresponding stereoisomers, solvates and pharmaceutically acceptable salts or esters thereof.

Preferred compounds of formula (I) of the present invention comprise combinations of the abovementioned preferred meanings.

More preferred compounds of formula (I) of the present invention comprise combinations of the abovementioned more preferred meanings.

Particularly preferred compounds of the formula (I) of the present invention comprise combinations of the abovementioned particularly preferred meanings.

The most preferred compounds of formula (I) of the present invention comprise combinations of the abovementioned most preferred meanings.

In a preferred embodiment, the compound of formula (I) of the present invention is a compound of formula (I-1) 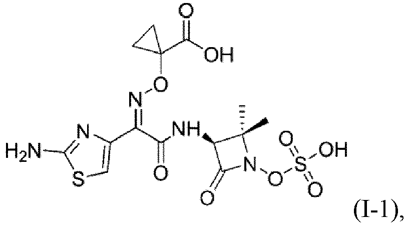 wherein R₁ represents H, R₂ represents H, R₃ and R₄ together form cyclopropyl, and Y is carboxyl.

In another preferred embodiment, the compound of formula (I) of the present invention is a compound of formula (I-2) 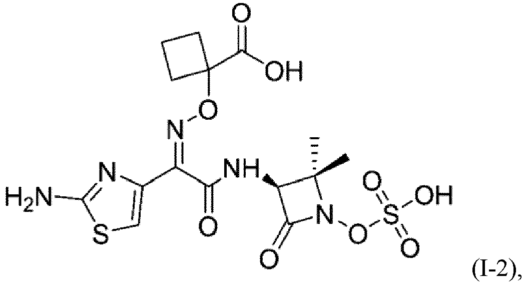 wherein R₁ represents H, R₂ represents H, R₃ and R₄ together form cyclobutyl, and Y is carboxyl.

In another preferred embodiment, the compound of formula (I) of the present invention is a compound selected from the following compounds:

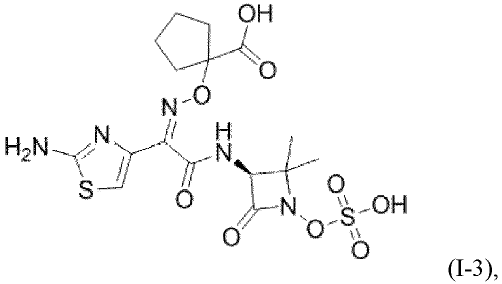

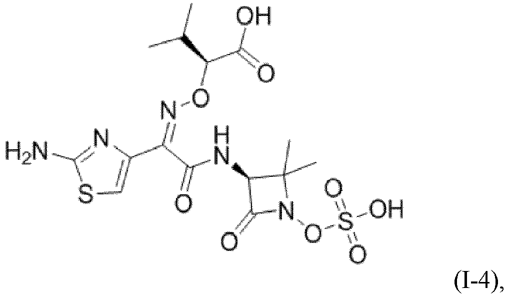

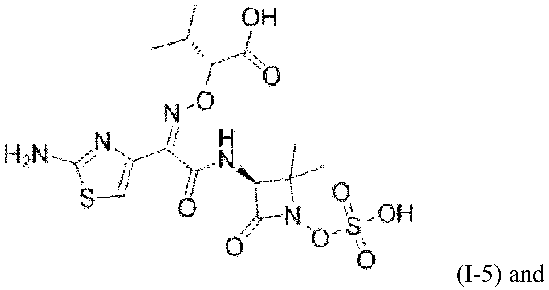

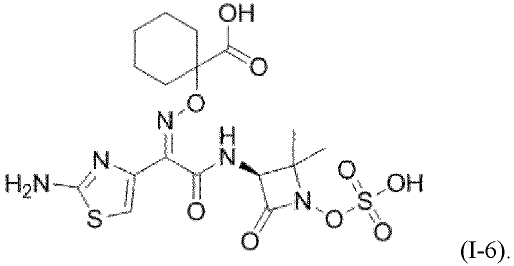

In addition, stereoisomers, solvates and pharmaceutically acceptable salts or esters of the compounds of formula (I) can be obtained by conventional means known to those skilled in the art.

Although the compounds of formula (I) and stereoisomers, solvates and pharmaceutically acceptable salts or esters thereof are exemplarily described herein, it will be apparent to those skilled in the art that these compounds are provided by way of illustration only and shall by no means limit the present invention.

### Preparation of the compound of formula (I)

The present invention also provides a method for preparing the aforementioned compound.

The compound of formula (I) is prepared by the scheme:

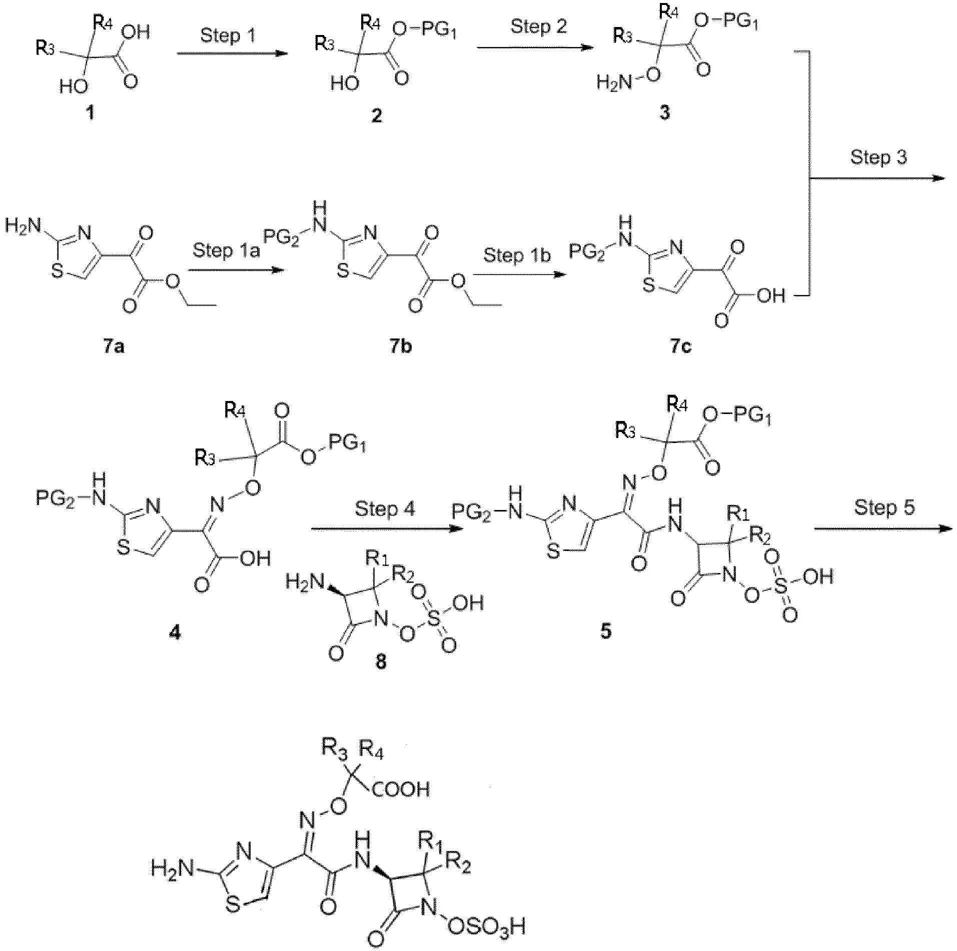

In the above formulae, PG₁ and PG₂ represent protecting groups; R₁, R₂, R₃, R₄ and Y are as previously defined

The carboxyl protecting group PG₁ is a carboxyl protecting group commonly used in the art. Not specifically defined, the "carboxyl protecting group" may be any one having its function. For example, it includes a lower alkyl group such as a methyl group, an ethyl group, a propyl group, an isopropyl group and a tert-butyl group; a halo-lower alkyl group such as a 2,2,2 trichloroethyl group; a lower alkenyl group such as an allyl group; and an aralkyl group such as a benzyl group, a p-methoxybenzyl group, a p-nitrosobenzyl group, a benzhydryl group and a trityl group; and is especially preferably a methyl group, an ethyl group, a tert-butyl group, an allyl group, a benzyl group, a p-methoxybenzyl group and a benzhydryl group. Those skilled in the art can make a reasonable selection according to actual needs. Preferably, PG₁ is -CHPh₂(benzhydryl).

The amino protecting group PG₂ is an amino protecting group commonly used in the art. Not specifically defined, the "amino or imino protecting group" may be any one having its function. For example, it includes -Boc(tert-butoxycarbonyl), -Cbz(benzyloxycarbonyl), - Teoc(trimethylsilylethoxycarbonyl), -Tos(p-toluenesulfonyl), -Trt(trityl) and -Bn(benzyl). Those skilled in the art can make a reasonable selection according to actual needs. Preferably, PG₂ is - Boc(tert-butoxycarbonyl).

The reaction conditions of the reaction are conventional conditions for the detachment of protecting groups, and vary according to PG₁ and PG₂, as described in A. G. Myers, J. Gleason, T. Yoon, D. W. Kung, J. Am. Chem. Soc., 1997, 119, 656; and M. Frankel, D. Ladkany, C. Gilon, Y. Wolman, Tetrahedron Lett., 1966, 7, 4765.

In the above step 1a, compound 7a is dissolved in a solvent, triethylamine is added, and triphenylchloromethane is added in batches; the mixture is reacted at room temperature. After the reaction is complete, an intermediate compound 7b is obtained.

Examples of the organic solvent used in the reaction in the above step 1a are, for example, dichloromethane, chloroform, toluene, tetrahydrofuran, N,N-dimethylformamide, acetonitrile, methanol, ethanol, isopropanol and a mixture thereof.

In the above step 1b, compound 7b is dissolved in 1,4-dioxane/water (50:50) and sodium hydroxide is added. The mixture is reacted under continuous stirring until the raw materials disappear. 1,4-dioxane is removed by evaporation under reduced pressure, and the pH is adjusted to 2-3. Stirring and filtration are performed. The filter cake is washed with water until the filtrate is neutral, and the filter cake is collected and dried to obtain compound 7c.

In the above step 1, compound 1 and diphenylbromomethane are dispersed in an organic solvent, and stirred at room temperature. DBU (1,8-diazabicyclo-undec-7-ene) is added, and the temperature is raised to 70-80°C. The reaction is performed under stirring until the raw materials are not further reduced. The temperature is reduced to room temperature. The reaction solution is extracted. The organic phases are combined, dried, and concentrated to obtain a residue. The residue is separated by column chromatography to obtain compound 2.

Examples of the organic solvent used in the reaction in the above step 1 include dichloromethane, toluene, tetrahydrofuran and N,N-dimethylformamide.

In the above step 2, compound 2 and diphenylphosphoryl hydroxylamine are dispersed in an organic solvent, purged with nitrogen 3-4 times, and stirred at 0°C. Subsequently, sodium tert-butoxide is added. The mixture is reacted under stirring at constant temperature until the reactants are completely converted. A saturated sodium chloride solution is added to the reaction solution, stirring is performed and the insoluble substances are removed by filtration to obtain compound 3.

Examples of the organic solvent used in the reaction in the above step 2 include dichloromethane, toluene, tetrahydrofuran and N,N-dimethylformamide.

In the above step 3, compound 7c is dispersed in an organic solvent, and a solution of compound 3 in methanol is added under stirring at room temperature. The mixture is reacted under stirring at constant temperature until the raw materials are fully converted. Column chromatography was performed to obtain compound 4.

Examples of the organic solvent used in the reaction in above step 3 include methanol, ethanol, isopropanol and N,N-dimethylformamide.

In the above step 4, compound 4 is dispersed in an organic solvent. HATU (2-(7-benzotriazole oxide)-N,N,N',N'-tetramethyluronium hexafluorophosphate), NaHCO3 and compound 8 are added respectively. The mixture is reacted under stirring at room temperature until the raw materials are fully converted. Column chromatography was performed to obtain compound 5.

Examples of the organic solvent used in the reaction in above step 4 include dichloromethane, toluene, tetrahydrofuran, and N,N-dimethylformamide.

In the above step 5, compound 5 is dissolved in an organic solvent and stirred at -5 to -10°C. Triethylsilane and trifluoroacetic acid are added thereto. The mixture is reacted at constant temperature until the reaction is completed. The solvent is removed by evaporation at room temperature under reduced pressure, and separation was performed to obtain the target product, i.e., the compound of general formula (I).

Examples of the organic solvent used in the reaction in the above step 5 include anhydrous dichloromethane, toluene, tetrahydrofuran and N,N-dimethylformamide.

Although the method for preparing the compound of formula (I) is exemplarily described herein, it will be apparent to those skilled in the art that this preparation method is provided by way of illustration only and shall by no means limit the present invention.

### Component (b)

The pharmaceutical composition of the present invention comprises (b) a lactamase inhibitor or an efflux pump inhibitor.

In one embodiment, the lactamase inhibitor is a diazabicyclooctanone β-lactamase inhibitor. In one embodiment, the diazabicyclooctanone β-lactamase inhibitor is in particular selected from the group consisting of avibactam, vaborbactam and relebactam.

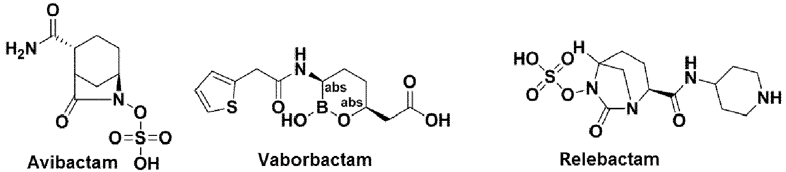

In one embodiment, the efflux pump inhibitor is selected from the group consisting of berberine, conessine and PaN:

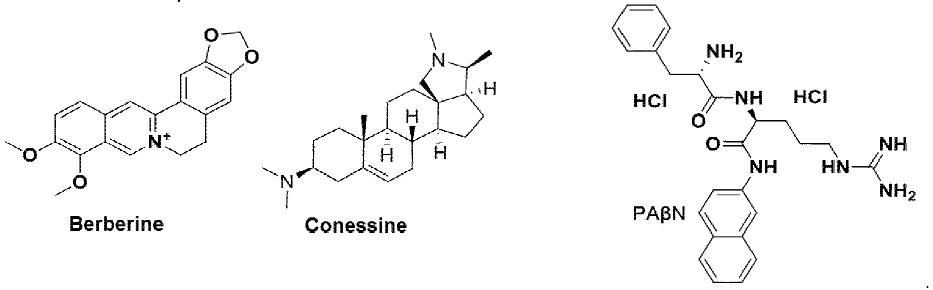

Although the compounds of component (b) are exemplarily described herein, it will be apparent to those skilled in the art that these compounds are provided by way of illustration only and shall by no means limit the present invention.

It has been surprisingly found that the pharmaceutical composition of the present invention has excellent antimicrobial effects, and as compared with other antimicrobial agents known in the prior art, exhibits stronger activities and can be used in smaller doses against the same microorganisms. Thus, the administration of the pharmaceutical composition of the present invention leads to fewer side effects on humans or animals, better tolerance, and less development of drug resistance.

The pharmaceutical composition of the present invention has excellent antimicrobial effects, especially for Gram-negative bacteria, such as *Escherichia coli, Klebsiella pneumoniae, Pseudomonas aeruginosa, Acinetobacter baumannii, Enterobacter cloacae, Enterobacter aerogenes, Salmonella typhi, Serratia marcescens, Citrobacter freundii, Providencia rettgeri, Proteus vulgaris, Proteus mirabilis, Pseudomonas maltophilia* and *Shigella flexneri,* and leads to low drug resistance.

In one embodiment, the bacteria are, for example, *Klebsiella pneumoniae, Escherichia coli* and *Enterobacter cloacae.* In another embodiment, the bacteria are *Acinetobacter baumannii* and *Pseudomonas aeruginosa.* Nevertheless, those skilled in the art should understand that these bacteria are only illustrative and in no way limit the present invention.

In a specific embodiment, the bacteria are, for example, the *Klebsiella pneumonia* strains ATCC2146, ATCC700603, CCPM(A)-0814R18, CCPM(A)-0814R33, CCPM(A)-081301, CCPM(A)-081705, CCPM(A)-081715, CCPM(A)-081729, CCPM(A)-081716 and CCPM(A)-0817R61, the *Escherichia coli* strains ATCC2469 and CCPM(A)-P-071301, and the *Enterobacter cloacae* strains CCPM(A)-P-11 1729 and ATCC2468. In another embodiment, the bacteria are the *Acinetobacter baumannii* stains ATCC 19606, ATCC 17978 and CCPM(A)-P-101633 (CRAB), and the *Pseudomonas aeruginosa* strains ATCC27853, PAO1 (CCPM(A)-P-09000032) and CCPM(A)-P-091626 (CRPA).

In one embodiment, the pharmaceutical composition of the present invention comprises at least one compound of formula (I), at least one β-lactamase inhibitor and/or at least one efflux pump inhibitor. In one embodiment, the pharmaceutical composition of the present invention comprises at least one compound of formula (I), at least one β-lactamase inhibitor selected from avibactam, vaborbactam and relebactam and/or at least one efflux pump inhibitor selected from berberine, conessine and PaβN.

In one embodiment, the pharmaceutical composition of the present invention comprises at least one compound selected from the compounds of formula (I-1), formula (I-2), formula (I-3), formula (I-4), formula (I-5) and formula (I-6), at least one β-lactamase inhibitor selected from avibactam, vaborbactam and relebactam and/or at least one efflux pump inhibitor selected from berberine, conessine and PaβN. In one embodiment, the pharmaceutical composition of the present invention comprises at least one compound selected from the compound of formula (I-1) and the compound of formula (I-2), at least one β-lactamase inhibitor selected from avibactam, vaborbactam and relebactam and/or at least one efflux pump inhibitor selected from berberine, conessine and PaβN. In one embodiment, the pharmaceutical composition of the present invention comprises the compound of formula (I-1) and at least one β-lactamase inhibitor selected from avibactam, vaborbactam and relebactam. In another embodiment, the pharmaceutical composition of the present invention comprises the compound of formula (I-1), and at least one efflux pump inhibitor selected from berberine, conessine and PaβN. In another embodiment, the pharmaceutical composition of the present invention comprises the compound of formula (I-2) and at least one β-lactamase inhibitor selected from avibactam, vaborbactam and relebactam. In another embodiment, the pharmaceutical composition of the present invention comprises the compound of formula (I-2), and at least one efflux pump inhibitor selected from berberine, conessine and PaβN.

In another embodiment, the pharmaceutical composition of the present invention comprises the compound of formula (I-1), at least one β-lactamase inhibitor selected from avibactam, vaborbactam and relebactam and at least one efflux pump inhibitor selected from berberine, conessine and PaβN. In another embodiment, the pharmaceutical composition of the present invention comprises the compound of formula (I-2), at least one β-lactamase inhibitor selected from avibactam, vaborbactam and relebactam and at least one efflux pump inhibitor selected from berberine, conessine and PaβN. The compounds of formula (I-1), formula (I-2), formula (I-3), formula (I-4), formula (I-5) and formula (I-6) here are defined as above.

In the pharmaceutical composition of the present invention, the compound of the present invention and a lactamase inhibitor or an efflux pump inhibitor exhibit a synergistic effect. The combined use of the compound of the present invention and a lactamase inhibitor or an efflux pump inhibitor reduces the minimum inhibitory concentrations of both the compound of the present invention and the lactamase inhibitor or the efflux pump inhibitor, with a fractional inhibitory concentration index (FICI) ≤ 0.5, indicating that the combination has a synergistic effect.

In one embodiment, the weight ratio of component (a) to component (b) is 1:0.3 to 1:10, preferably 1:0.5 to 1:7, more preferably 1:0.8 to 1:6, most preferably 1:1 to 1:5.

The optimal dose and interval of administration of the compound of the present invention or the pharmaceutical composition of the present invention are determined by the properties of the compound and the external conditions such as the form, route and site of administration, and the particular mammals to be treated. This optimal dose can be determined by conventional techniques. The optimal course of treatment, i.e., the daily dose of the compound of the present invention or the pharmaceutical composition of the present invention within a specific time, can be determined by methods known in the art.

In one embodiment, the dose of the pharmaceutical composition of the present invention in a single administration is 1 to 5000 mg active ingredient/kg body weight, preferably 2 to 4000 mg active ingredient/kg body weight, more preferably 5 to 3000 mg active ingredient/kg body weight, particularly preferably 10 to 1000 mg active ingredient/kg body weight, further preferably 13 to 500 mg active ingredient/kg body weight, e.g., 14 to 300 mg active ingredient/kg body weight, and 15 to 200 mg active ingredient/kg body weight, most preferably 15 to 100 mg active ingredient/kg body weight. In one embodiment, the compound of the present invention is administered at least once a day, e.g., once, twice, three, four or five times a day. Preferably, the compound of the present invention is administered once, twice or three times daily. The term "active ingredient" here refers to the compound of the present invention and a lactamase inhibitor or an efflux pump inhibitor.

The pharmaceutical composition of the present invention further optionally comprises a pharmaceutically acceptable carrier, an excipient and/or other adjuvants. When the pharmaceutical composition comprises a pharmaceutically acceptable carrier, an excipient and/or other adjuvants, an effective dose of the pharmaceutical composition of the present invention is usually combined with one or more pharmaceutically acceptable carriers, excipients and/or other adjuvants to prepare suitable administration or dosage forms, a procedure involving mixing, granulating, compressing or dissolving the components by suitable means. The content of the carrier in the pharmaceutical composition can be 1 to 98% by weight, usually about 80% by weight. For convenience, local anesthetic, preservatives, buffers and other adjuvants can be directly dissolved in the carrier. Therefore, the present invention provides a pharmaceutical composition comprising the compound of formula (I) of the present invention, or a stereoisomer, a solvate or a pharmaceutically acceptable salt or an ester thereof, and a pharmaceutically acceptable carrier, an excipient and/or other adjuvants.

The present invention also provides the use of the pharmaceutical composition of the present invention as an antimicrobial agent. In the use invention, the term "microorganism" has the same meaning as in the description concerning the pharmaceutical composition.

In addition, the present invention also provides the use of the pharmaceutical composition of the present invention in the manufacture of a medicament for treating infectious diseases.

In addition, the present invention also provides a method for treating infectious diseases, which comprises administering component (a) and component (b) to humans or animals. Here, component (a) and component (b) have the meanings as described above. In one embodiment, component (a) and component (b) are pharmaceutical forms separately prepared. In one embodiment, the interval between administration of component (a) and component (b) in the method of the present invention is no more than 4 hours, preferably no more than 3 hours, more preferably no more than 2 hours, even more preferably no more than 1 hour, particularly preferably no more than 30 minutes; most preferably, component (a) and component (b) are administered simultaneously, for example, in the form of a pharmaceutical composition comprising the two components , i.e., in the form of the composition of the present invention.

In one embodiment, the method for treating infectious diseases of the present invention comprises administering the pharmaceutical composition of the present invention to humans or animals.

### Administration

The pharmaceutical composition of the present invention can be administered according to any of the following routes: oral, spray inhalation, rectal, nasal, vaginal, topical, parenteral such as subcutaneous, intravenous, intramuscular, intraperitoneal, intrathecal, intraventricular, intrasternal or intracranial injection or infusion, or by means of an explanted reservoir, wherein oral, intramuscular, intraperitoneal or intravenous administration route is preferred.

The pharmaceutical composition of the present invention can be administered in unit dosage form. The dosage form may be a liquid dosage form or a solid dosage form. The liquid dosage form can be true solutions, colloids, particulates, or suspensions. Other dosage forms include, for example, tablets, capsules, pills, aerosols, pills, powders, solutions, suspensions, emulsions, granules, suppository, lyophilized powder for injection, clathrate, implants, patches, liniment and sustained-release preparations.

Oral tablets and capsules can be prepared by using methods well known in pharmaceutical chemistry. Tablets can be coated.

Oral liquid can be made into water and oil suspensions, solutions, emulsions, or syrups, or can also be made into dry preparation, to which, before use, water or other suitable medium is added. If necessary, a flavoring or coloring agent can be added.

Suppositories can contain conventional suppository bases, for example, cacao butter or other glycerides.

For parenteral administration, the liquid dosage form is usually made of the pharmaceutical composition of the present invention and a sterilized carrier. The carrier is preferably water. According to the difference of the selected carrier and of the concentration of the drug, the compound can be dissolved in the carrier and can also be made into a suspension. When being made into a solution for injection, the pharmaceutical composition is firstly dissolved in water, and then filtered and sterilized before being packaged into a sealed bottle or an ampoule.

The pharmaceutical composition of the present invention can also be made into sterile preparations for injection, including crystalline powder for injection, lyophilized powder for injection, etc.

For topical administration on skin, the pharmaceutical composition of the present invention can be made into an appropriate form of ointments, lotions, or creams, wherein the active ingredient is suspended or dissolved in one or more carriers.

The present invention is described in more detail below by way of examples.

### Preparation Example

### Preparation Example 1: Synthesis of a compound of formula (I-1)

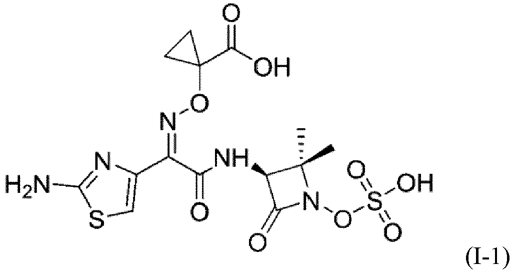

Step 1a: Compound 7a (1.0 mmol) was dissolved in DMF (10.0 v/g), triethylamine (2.0 mmol) was added, and triphenylchloromethane (1.2 mmol) was added in batches. The mixture was reacted under stirring at room temperature for 6 h; after the reaction was complete as monitored by TLC, water/ethyl acetate was added for extraction. The organic phases were combined, dried and concentrated. A column chromatography separation was performed to obtain the target compound 7b.

Step 1b: Compound 7b (1.0 mmol) was dissolved in 1,4-dioxane (5 v/g)/water (5 v/g), and stirred at room temperature, and sodium hydroxide (5.0 mmol) was added. The mixture was reacted under continuous stirring. After the raw materials disappeared as monitored by TLC, 1,4-dioxane was removed by evaporation under reduced pressure. The pH was adjusted to 2-3. Stirring was performed for 10 minutes before filtration. The filter cake was washed with water until the filtrate was neutral. The filter cake was collected and dried to obtain compound 7c.

Step 1: 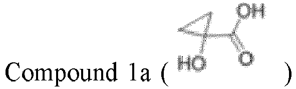 (1.0 mmol) and diphenylbromomethane (1.1 mmol) were dispersed in toluene solution (10.0 v/g), and stirred at room temperature. DBU (1.5 mmol) was added. The temperature was raised to 70-80°C, and the reaction was performed with stirring for 12-16 hours. After the raw materials were not further reduced as monitored by TLC, the temperature was lowered to room temperature. Water (10.0 v/g x 2 ) was added to extract the reaction solution and the aqueous layer was backwashed with ethyl acetate (10 v/g x 2 ). The organic phases were combined, dried, and concentrated to obtain a residue, and the residue was separated by column chromatography to obtain compound 2a as a transparent oil (post-cured) [1H NMR (400 MHz, CDCl3) δ(ppm) 7.42 - 7.31 (m, 10H), 6.96 (s, 1H), 1.44 (dd, J = 8.1, 4.9 Hz, 2H), 1.25 (dd, J = 8.1, 4.9 Hz, 2H)].

Step 2: Compound 2a (1.0 mmol) and diphenylphosphoryl hydroxylamine (1.1 mmol) was dispersed in an anhydrous tetrahydrofuran solution (10.0 v/g), purged with nitrogen 3-4 times, and stirred at 0°C. Subsequently, sodium tert-butoxide (1.5 mmol) was added. The reaction was performed under stirring at constant temperature for 1-2 hours. After the reactants were completely converted as monitored by TLC, a saturated sodium chloride solution (5.0 v/g) was added to the reaction solution, stirring was performed for 30 min and then the insoluble substances were removed by filtration. The filter cake was washed with ethyl acetate. Ethyl acetate (10.0 v/g) and water (5.0 v/g) were added to the filtrate, and liquid phase separation was performed. The organic phase was washed once with water. The aqueous phases were combined, and backwashed with ethyl acetate. The organic phase was combined, dried and concentrated to give 3a as an oil [1H NMR (600 MHz, DMSO) δ(ppm) 7.46 -7.27 (m, 10H), 6.84 (s, 1H), 6.36 (s, 2H), 1.30 - 1.22 (m, 4H)].

Step 3: Compound 7c was dispersed in methanol (10.0 v/g). Under stirring at room temperature, a solution of compound 3a in methanol was added. The reaction was performed under stirring at constant temperature for 30 minutes. After the raw materials were completely converted as monitored by TLC, the solvent was removed by evaporation under reduced pressure. Column chromatography separation was performed to obtain compound 4a as an off-white solid [1H NMR (600 MHz, DMSO) δ(ppm) 8.84 (s, 1H), 7.51 -7.15 (m, 25H), 6.86 (s, 2H), 1.48 - 1.28 (m, 4H)].

Step 4: Compound 4a (1.0 mmol) was dissolved in DMF (10.0 v/g) and stirred at room temperature, and HATU (1.2 mmol), NaHCO₃ (2.0 mmol) and compound 8 (1.3 mmol) were added respectively. The mixture was reacted under continuous stirring at constant temperature for 12 hours until the raw materials disappeared as monitored. Ethyl acetate (10.0 v/g) was added for dilution, washing was performed twice with water, and backwashing was performed once with ethyl acetate. The organic phases were combined, dried, concentrated. Column chromatography separation was performed to obtain compound 5a as an off-white foamy solid [1H NMR (600 MHz, DMSO) δ(ppm) 9.29 (d, J = 7.9 Hz, 1H), 8.91 (s, 1H), 7.45 -7.43 (m, 3H), 7.36 - 7.33 (m, 6H), 7.32 -7.22 (m, 15H), 6.86 (s, 1H), 6.76 (d, J = 0.5 Hz, 1H), 4.56 (d, J =7.9 Hz, 1H), 1.50 - 1.40 (m, 5H), 1.30 (m, 2H), 1.20 (s, 2H)].

Step 5: The aforementioned white solid 5a (1.0 mmol) was dissolved in anhydrous dichloromethane (10.0 v/g), and stirred at -5 to -10°C. Triethylsilane (2.0 mmol) and trifluoroacetic acid (100.0 mmol) were added thereto. The mixture was reacted at constant temperature for 5-6 hours until the reaction was completed as monitored by TLC. The solvent was removed by evaporation under reduced pressure at room temperature. Ethyl acetate was added to the residue, stirring was performed at room temperature for 1 hour, and filtration was performed. The filter cake was washed 3 times with ethyl acetate, collected and dried. The above solid was dissolved in methanol/water, and preparation and isolation by pre-HPLC (YMC ODS-A, 5 um, 10*250 mm, 2.5 mL/min, 2%-50% acetonitrile/0.1% formic acid solution) were performed to obtain the target product (I-1).

¹H NMR (600 MHz, DMSO) δ(ppm) 9.40 (d, *J* = 7.9 Hz, 1H), 6.89 (s, 1H), 4.62 (d, *J* = 8.0 Hz, 1H), 1.44 (s, 3H), 1.36 (m, 4H), 1.25 (s, 3H). 13C NMR (151 MHz, DMSO) δ(ppm) 173.7, 169.8, 161.9, 111.6, 68.3, 63.3, 61.3, 60.2, 23.8, 21.0, 16.2. ¹³C NMR (151 MHz, DMSO) δ 173.7, 169.8, 161.9, 111.6, 68.3, 63.3, 61.3, 60.2, 23.8, 21.0, 16.2. HRMS: calculated for C₁₄H₁₇N₅O₉S₂ [M-H] 462.0396, found 462.0394

### Preparation Example 2: Synthesis of a compound of formula (I-2)

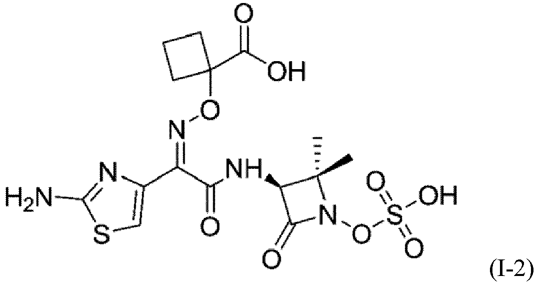

As for the method for synthesizing the compound of formula (I-2), please refer to the synthesis of the compound of formula (I-1).
2b: ¹H NMR (600 MHz, DMSO) δ(ppm) 7.44 (d, *J* = 7.4 Hz, 4H), 7.37 (t, *J* = 7.6 Hz, 4H), 7.29 (t, *J* = 7.3 Hz, 2H), 6.83 (s, 1H), 5.87 (s, 1H), 2.47 - 2.40 (m, 2H), 2.16 - 2.07 (m, 2H), 1.89 - 1.74 (m, 2H).;
3b: ¹H NMR (600 MHz, DMSO) δ(ppm) 7.35 (ddd, J = 46.9, 31.4, 7.3 Hz, 10H), 6.85 (s, 1H), 6.11 (s, 2H), 2.35 (ddd, J = 13.4, 7.2, 3.7 Hz, 2H), 2.22 - 2.09 (m, 2H), 1.92 - 1.74 (m, 2H).;
4b: ¹H NMR (600 MHz, DMSO) δ(ppm) 8.58 (s, 1H), 7.50 -7.15 (m, 25H), 6.78 (s, 1H), 6.12 (s, 1H), 2.42 (s, 2H), 2.25 - 2.22 (m, 2H), 1.96 - 1.69 (m, 2H). (containing 50% impurities; see Z21e);

The compound of Formula (I-2): ¹H NMR (600 MHz, DMSO) δ(ppm) 9.56 (d, J = 7.3 Hz, 1H), 6.89 (s, 1H), 4.66 (d, J = 7.8 Hz, 1H), 2.45 (s, 2H), 2.35 -2.19 (m, 2H), 1.99 - 1.79 (m, 2H), 1.46 (s, 3H), 1.32 (s, 3H). ¹³C NMR (151 MHz, DMSO) δ(ppm) 173.6, 170.3, 161.8, 129.5, 128.8, 126.7, 111.5, 83.7, 68.4, 61.4, 30.6, 30.5, 23.7, 21.0, 13.9. HRMS: calculated for C₁₅H₁₉N₅O₉S₂ [M+Na] 500.0516, found 500.0500.

The compounds of Formula (I-3) to Formula (I-6) were prepared using suitable raw materials with reference to the method in Preparation Example 1.

The compound of Formula (I-3): ¹H NMR (600 MHz, DMSO) δ (ppm) 9.51 (d, J = 7.6 Hz, 1H), 6.86 (s, 1H), 4.63 (d, J = 7.7 Hz, 1H), 2.09-1.97 (m, 4H), 1.73 -1.64 (m, 4H), 1.46 (s, 3H), 1.29 (s, 3H). 13C NMR (151 MHz,DMSO) δ(ppm) 174.83, 163.48, 161.82, 149.85, 140.60, 110.92,92.84, 68.28, 61.44, 35.83, 24.64, 24.51, 23.75, 20.91. HRMS: calculated for C₁₆H₂₁N₅O₉S₂ [M-H] 490.0708, found 490.0700.

The compound of Formula (I-4): ¹H NMR (600 MHz, DMSO) δ(ppm) 9.58 (d, J = 7.5 Hz, 1H), 6.89 (s, 1H), 4.65 (d, J = 7.6 Hz, 1H), 4.37 (d, J = 5.2 Hz, 1H), 2.13 - 2.07 (m, 1H), 1.45 (s, 3H), 1.30 (s, 3H), 1.00 - 0.86 (m,6H). 13C NMR (151 MHz, DMSO) δ172.2, 170.2, 164.4, 163.5, 161.8, 11.7, 111.4, 87.6, 68.1, 61.4, 30.1, 23.8, 21.1, 18.8, 18.2. HRMS: calculated for C₁₅H₂₁N₅O₉S₂ [M+Na] 502.0673, found 502.0677

The compound of Formula (I-5): 1H NMR (600 MHz, DMSO) δ 9.53 (d, J = 7.4 Hz, 1H), 6.87 (s, 1H), 4.62 (d, J = 7.5 Hz, 1H), 4.31 (d, J = 5.7 Hz, 1H), 2.08 (dd, J= 13.1, 6.6 Hz, 1H), 1.45 (s, 3H), 1.30 (s, 3H), 0.96 (dd, J= 15.5,6.7 Hz, 6H). 13C NMR (151 MHz, DMSO) δ172.4, 169.9, 166.0, 163.6, 161.8, 111.2, 110.1, 87.6, 68.2, 61.4, 30.2, 23.9, 20.9, 19.0, 18.3. HRMS: calculated for C₁₅H₂₁N₅O₉S₂ [M+Na] 502.0673, found 502.0661

The compound of Formula (I-6): 1H NMR (600 MHz, DMSO) δ 9.37 (s, 1H), 6.72 (s, 1H), 4.60 (d, 1H), 4.22 (s, 1H), 2.02 (s, 1H), 1.43 (s, 3H), 1.29 (s, 3H), 0.93 (s, 6H). HRMS: calculated for C_{17H}23N₅O₉S2 [M-H] 504.0864, found 504.0850.

### Effect Examples

Test samples: Compounds I-1 and I-2, avibactam, vaborbactam, relebactam, and berberine, conessine and PaβN,
wherein avibactam and berberine were purchased from Innochem Technology Co., Ltd., vaborbactam and relebactam were purchased from Shanghai Macklin Biochemical Technology Co., Ltd., conessine was purchased from J&K Scientific Co., Ltd., and PaβN was purchased from MedChemExpress.

Test strains: the test strains were from the American Type Culture Collection (ATCC) and the Collection Center of Pathogen Microorganisms of the Chinese Academy of Medical Sciences-Division for Medicinal Microorganisms Related Strains (CAMS-CCPM-A) (these strains (see Tables 1 to 5 for details) are available to the public from this Collection Center).

### Methods for evaluating the antimicrobial activities of individual compounds (MIC assay)

Mueller-Hinton broth (MH broth) medium, Trypticase Soy Broth (TSB Broth) medium, purchased from BD Difco, Cockeysville, MD, USA;
96-well plate: Corning Costar, Cambridge, MA, USA;

Test method: the antibacterial activities were tested by the microdilution method according to the CLSI/NCCLS standard.
(1) Preparation of the test samples: 4 mg of each sample was taken, and was prepared into a test solution at 1,024 µg/mL with MH broth, and the test solution was diluted two-fold in a 96-well plate to prepare solutions of a series of gradient concentrations, the volume in each well being 90 µL.
(2) Preparation of a bacteria solution: The test strains were amplified by Trypticase Soy Broth (TSB Broth) according to the CLSI/NCCLS standard.
(3) Addition of the samples: a bacteria solution with a concentration of 6×10⁵ cfu/mL was prepared, and 10 µL of the bacteria solution was pipetted into each well of the 96-well plate. Three parallel experiments were set up for each concentration and the results were averaged. To the negative control group, 10 µL of the bacterial solution and 90 µL MH broth were added.
(4) Incubation: the 96-well plate was placed in a 37°C constant-temperature incubator for 18 hours, and the growth of the strains was observed. The drug concentration in the well with no microbial growth was recorded as the MIC.

### Research methods for combined in vitro use of the drugs

Mueller-Hinton broth (MH broth) medium, Trypticase Soy Broth (TSB Broth) medium, purchased from BD Difco, Cockeysville, MD, USA;
96-well plate: Corning Costar, Cambridge, MA, USA;

Test method: the checkerboard dilution method was used for determination.
(1) Preparation of the test samples: 4 mg of each sample was taken and was prepared into a test solution at 2,048 µg/mL with MH broth, and the test solution was diluted two-fold in a 96-well plate to prepare solutions of a series of gradient concentrations.
(2) Preparation of a bacteria solution: The test strains were amplified by Trypticase Soy Broth (TSB Broth) according to the CLSI/NCCLS standard.
(3) Addition of the samples: 10 µL of the bacteria solution with a concentration of 6×10⁵ CFU/mL was pipetted into each well of the 96-well plate. Each well contained the antibacterial drugs (the compound of formula (I-1) and the compound of formula (I-2)) diluted two-fold with broth and the aforementioned lactamase inhibitor or efflux pump inhibitor. The actual concentrations of the samples of the compound of formula (I-1) and compound of formula (I-2) were made to be 64.0, 32.0, 16.0, 8.0, 4.0, 2.0, 1.0, and 0.0 µg/mL respectively, and the actual concentrations of the samples of the lactamase inhibitor or efflux pump inhibitor were made to be 256, 128, 64, 32.0, 16.0, 8.0, 4.0, 2.0, 1.0, 0.5, 0.25 and 0.0 µg/mL respectively. The samples were mixed thoroughly. Three parallel experiments were set up for each concentration and the results were averaged. To the negative control group, 10 µL of the bacterial solution and 90 µL MH broth were added.
(4) Incubation: the 96-well plate was placed in a 37°C constant-temperature incubator for 18 hours, and the growth of the strains was observed.
(5) Calculation: The *in vitro* effect of each drug-inhibitor combination was analyzed by calculating the FICI value: FICI = (MIC of drug A in the combination/MIC of drug A alone) + (MIC of drug B in the combination/MIC of drug B alone). A FICI of ≤ 0.5 means that the two drugs had good synergistic effect; when 0.5 < FICI < 4, it means that the two drugs showed no synergistic effect; and a FICI of ≥ 4 means that the two drugs had antagonistic effects.

In order to ensure the reliability of the data, three repeated tests were conducted on different dates and the results were averaged.

### Effect Example 1

According to the assay method described above, the MICs of each of the compound of formula (I-1), the compound of formula (I-2) and the β-lactamase inhibitors against *Enterobacteriaceae* were determined. The test results are shown in Table 1.

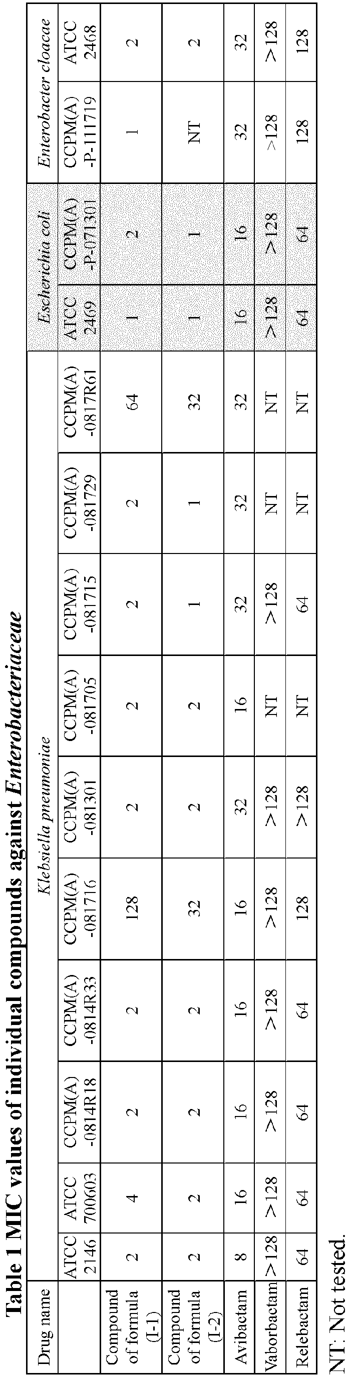

As can be seen from Table 1, against some drug-resistant *Enterobacteriaceae,* such as the *Klebsiella pneumoniae* strains ATCC 2146, ATCC700603, CCPM(A)-0814R18, CCPM(A)-0814R33, CCPM(A)-081705, CCPM(A)-081715 and CCPM(A)-081729, the *Escherichia coli* strains ATCC2469 and CCPM(A)-P-071301, and the *Enterobacter cloacae* strains 17-19 and ATCC2468, the MICs of the compound of formula (I-1) and the compound of formula (I-2) were between 0.5 and 4 µg/mL respectively; the MICs of the compound of formula (I-1) and the compound of formula (I-2) against the *Klebsiella pneumoniae* strains CCPM(A)-081716 and CCPM(A)-0817R61 were between 32 and 128 µg/mL respectively. The β-lactamase inhibitors avibactam, vaborbactam and relebactam themselves had relatively weak activities against the aforementioned *Enterobacteriaceae.*

### Effect Example 2

According to the checkerboard assay method described above, the MICs of the compound of formula (I-1) and formula (I-2) each in combination with a β-lactamase inhibitor against *Enterobacteriaceae* was determined. The test results are shown in Tables 2A to 2C and Table 3. Tables 2A to 2C show the data on the antibacterial spectrum of the compound of formula (I-1) combined with avibactam, vaborbactam and relebactam respectively. Table 3 shows the data on the antibacterial spectrum of the compound of formula (I-2) combined with avibactam.

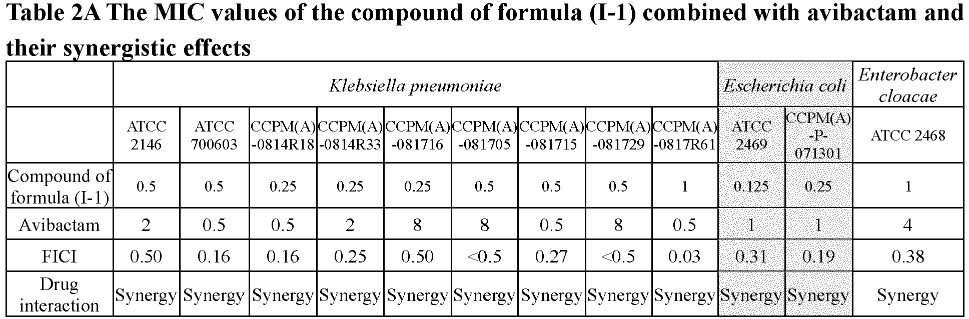

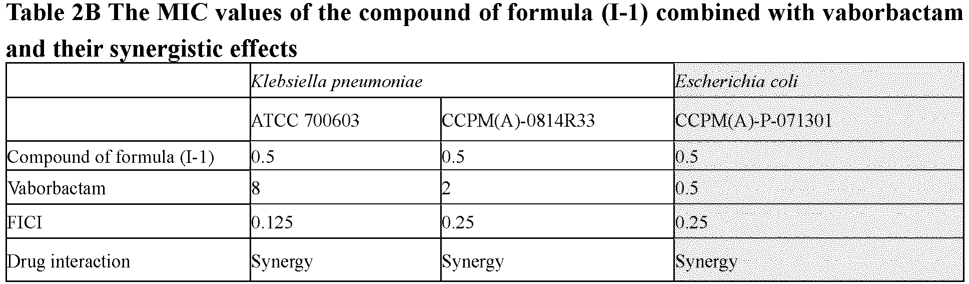

**Table 2C The MIC values of the compound of formula (I-1) combined with vaborbactam and their synergistic effects**

| | *Klebsiella pneumoniae* | |
|---|---|---|
| | ATCC 2146 | ATCC 700603 |
| Compound of formula (I-1) | 0.5 | 0.125 |
| Relebactam | 0.5 | 8 |
| FICI | 0.26 | 0.16 |
| Drug interaction | Synergy | Synergy |

**Table 3 The MIC values of the compound of formula (I-2) combined with avibactam and their synergistic effects**

| | *Klebsiella pneumoniae* | | | |
|---|---|---|---|---|
| | ATCC 2146 | ATCC 700603 | CCPM(A)-081715 | CCPM(A)-081729 |
| Compound of formula (I-2) | 0.5 | 0.5 | 0.5 | 0.5 |
| Avibactam | 0.5 | 0.5 | 4 | 4 |
| FICI | 0.31 | 0.14 | 0.5 | 0.375 |
| Drug interaction | Synergy | Synergy | Synergy | Synergy |

After the compound of formula (I-1) was combined with avibactam, the MIC values of the former against all of the 12 drug-resistant *Enterobacteriaceae* strains tested were significantly reduced, and the combination showed synergictic effects (FICI < 0.5). After the compound of formula (I-1) was combined with vaborbactam and relebactam respectively, the MIC values of the former against some drug-resistant *Enterobacteriaceae* strains were reduced, and both combinations showed synergictic effects (FICI < 0.5). This shows that the compound preparation comprising the compound of formula (I-1) and a diazabicyclooctanone β-lactamase inhibitor, such as avibactam, vaborbactam and relebactam, has great potential to be developed into a medicament for clinical treatments for multidrug-resistant Gram-negative *Enterobacteriaceae* infections.

After the compound of formula (I-2) was combined with avibactam, the MIC values of the former against all of the 4 drug-resistant *Enterobacteriaceae* strains tested were significantly reduced, and the combination showed synergictic effects (FICI < 0.5). This shows that the compound preparation comprising the compound of formula (I-2) and a β-lactamase inhibitor, such as avibactam, also has great potential to be developed into a medicament for clinical treatments for multidrug-resistant Gram-negative *Enterobacteriaceae* infections.

### Effect Example 3

According to the assay method described above, the individual MICs of each of the compound of formula (I-1) and the efflux pump inhibitors against *Acinetobacter baumannii* or *Pseudomonas aeruginosa* were determined. The test results are shown in Table 4.

**Table 4 MIC values of individual compounds against Acinetobacter baumannii or Pseudomonas aeruginosa**

| | *Acinetobacter baumannii* | | | *Pseudomonas aeruginosa* | | |
|---|---|---|---|---|---|---|
| | ATCC 19606 | ATCC 17978 | CCPM(A)-P -101633(CRAB) | ATCC27853 | PAO1(CCPM(A)-P -09000032) | CCPM(A)-P -091626(CRPA) |
| Compound of formula (I-1) | 16 | 16 | 32 | 16 | 16 | 32 |
| Berberine | >1024 | >1024 | >1024 | >1024 | >1024 | >1024 |
| Conessine | >256 | NT | NT | NT | >256 | NT |
| PAβN | >256 | >256 | >256 | >256 | >256 | >256 |

As can be seen from Table 4, against various *Acinetobacter baumannii* or *Pseudomonas aeruginosa* strains, the MICs of the compound of formula (I-1) were between 16 and 32 µg/mL. The efflux pump inhibitors berberine, conessine and PAβN showed relatively weak activities against the aforementioned various *Acinetobacter baumannii* or *Pseudomonas aeruginosa* strains.

### Effect Example 4

According to the assay method described above, the MICs of the compound of formula (I-1) combined with an efflux pump inhibitor selected from berberine, conessine and PAβN against the bacteria were determined. The test results are shown in Tables 5A to 5C.

**Table 5A The MIC values of the compound of formula (I-1) combined with berberine and their synergic effects**

| Synergistic effects of the compound of formula (I-1) and berberine | | | | | | |
|---|---|---|---|---|---|---|
| | *Acinetobacter baumannii* | | | *Pseudomonas aeruginosa* | | |
| | ATCC 19606 | ATCC 17978 | CCPM(A)-P -101633(CRAB) | ATCC27853 | PAO1(CCPM(A)-P -09000032) | CCPM(A)-P -091626(CRPA) |
| Compound of formula (I-1) | 4 | 4 | 16 | 16 | 16 | 32 |
| Berberine | 32 | 256 | >0.5 | * | * | * |
| FICI | <0.28 | <0.5 | >0.5 | >1 | >1 | >1 |

| | | | | | | |
|---|---|---|---|---|---|---|
| * indicates that no synergistic effect was observed within the tested concentration range | | | | | | |

**Table 5B The MIC values of the compound of formula (I-1) combined with conessine and their synergistic effects**

| Synergistic effects of the compound of formula (I-1) and conessine | | | | | | |
|---|---|---|---|---|---|---|
| | *Acinetobacter baumannii* | | | *Pseudomonas aeruginosa* | | |
| | ATCC 19606 | ATCC 17978 | CCPM(A)-P -101633(CRAB) | ATCC27853 | PAO1(CCPM(A)-P -09000032) | CCPM(A)-P -091626(CRPA) |
| Compound of formula (I-1) | 4 | NT | NT | NT | 16 | NT |
| Conessine | 4 | NT | NT | NT | >256 | NT |
| FICI | <0.28 | NT | NT | NT | >1 | NT |

| | | | | | | |
|---|---|---|---|---|---|---|
| NT: Not tested. | | | | | | |

**Table 5C The MIC values of the compound of formula (1-1) combined with PAβN and their synergistic effects**

| Synergistic effects of the compound of formula (I-1) and PAβN | | | | | | |
|---|---|---|---|---|---|---|
| | *Acinetobacter baumannii* | | | *Pseudomonas aeruginosa* | | |
| | ATCC 19606 | ATCC 17978 | CCPM(A)-P -101633(CRAB) | ATCC27853 | PAO1(CCPM(A)-P -09000032) | CCPM(A)-P -091626(CRPA) |
| Compound of formula (I-1) | 16 | 16 | 16 | 2 | 2 | 8 |
| PAβN | * | * | >0.5 | 64 | 64 | 128 |
| FICI | >1 | >1 | >0.5 | <0.375 | <0.375 | ≤0.5 |

| | | | | | | |
|---|---|---|---|---|---|---|
| * indicates that no synergistic effect was observed within the tested concentration range | | | | | | |

After the compound of formula (I-1) was combined with berberine, the MIC values of the former against the 2 drug-resistant *Acinetobacter baumannii* strains ATCC 19606 and ATCC 17978 were reduced four-fold, and the combination of the compound of formula (I-1) and berberine showed synergictic effects (FICI < 0.5). After the compound of formula (I-1) was combined with conessine, the MIC values of the former against the drug-resistant *Acinetobacter baumannii* strain ATCC19606 tested were reduced four-fold, and the combination of the compound of formula (I-1) and conessine showed synergictic effects (FICI < 0.5).

After the compound of formula (I-1) was combined with PAβN, the MIC values of the former against the 2 drug-resistant *Pseudomonas aeruginosa* strains ATCC27853 and PAO1(CCPM(A)-P-09000032) were reduced eight-fold, and the combination showed synergictic effects (FICI < 0.5), and the MIC value against the drug-resistant *Pseudomonas aeruginosa* strain CCPM(A)-P-091626(CRPA) was reduced four-fold, and the combination of the compound of formula (I-1) and PAβN likewise showed synergictic effects (FICI < 0.5).

The above results show that the compound preparation comprising the compound of formula (I-1) and berberine or conessine has the potential to be developed into a medicament for clinical treatments for *Acinetobacter baumannii* infections, and the compound preparation comprising the compound of formula (I-1) and PaβN has great potential to be developed into a medicament for clinical treatments for *Pseudomonas aeruginosa* infections.

### Effect Example 5: Study on the combined in vivo use of the drugs

ICR mice (half male and half female) with healthy appearance and a weight of 18 to 22 g were used. The mice were randomly grouped, with 5 mice (either male or female) in each dose group. A test bacteria solution containing NDM-1-producing *Klebsiella pneumoniae* was diluted with 5% highly active dry yeast, and 0.5 mL of the bacteria solution (the 100% minimum lethal dose (100% MLD, 3.0×10⁶ CFU/mouse)) was intraperitoneally injected into the mice.

To the mice infected intraperitoneally with NDM-1-producing *Klebsiella pneumoniae* (at a dose of 3.0×10⁶ CFU), drugs were administered once through the tail vein at certain doses (see Table 5) at 1 h and 6 h respectively after the infection for a total of two administrations, with the volume of the drugs administered being 10 mL/kg. The survival of the animals in each group was observed within 7 days, and the survival rates of the mice were calculated. The control group was not given any drugs, and given only physiological saline, and the seven-day survival rate of the mice was 0.

The protective effects of the compound of formula (I-1) administered alone and in combination with avibactam on mice systemically infected with *Klebsiella pneumoniae* ATCC BAA2146 (NDM-1) are shown in Table 5. It is well known to those skilled in the art that avibactam itself has no *in vivo* antibacterial effect on this test strain.

**Table 5 In vivo test on the effect of intravenous injection of the compound of formula (I-1) and avibactam on mice intraperitoneally infected with Klebsiella pneumoniae ATCC BAA2146 (NDM-1)**

| Ratio | Drug name | Dose (mg/kg) | Logarithmic dose (log) | Number of animals | Number of animals survived | Survival rate of animals (%) |
|---|---|---|---|---|---|---|
| Individual compound | Compound of formula (I-1) | 32 | 1.51 | 5 | 3 | 60 |
| | Compound of formula (I-1) | 16 | 1.20 | 5 | 1 | 20 |
| Combinations (1:4) | Compound of formula (I-1)+avibactam | 32:128 | 1.51:2.11 | 5 | 5 | 100 |
| | Compound of formula (I-1)+avibactam (1:4) | 16:64 | 1.20:1.81 | 5 | 5 | 100 |
| Combinations (1:2) | Compound of formula (I-1)+avibactam | 32:64 | 1.51:1.81 | 5 | 5 | 100 |
| | Compound of formula (I-1)+avibactam | 16:32 | 1.20:1.51 | 5 | 5 | 100 |
| Combinations (1:1) | Compound of formula (I-1)+avibactam | 32:32 | 1.51:1.51 | 5 | 5 | 100 |
| | Compound of formula (I-1)+avibactam | 16:16 | 1.20:1.20 | 5 | 5 | 100 |
| | Control group | - | - | 5 | 0 | 0 |

As shown from Table 5, after injecting the compound of formula (I-1) to the mice infected intraperitoneally with NDM-1-producing *Klebsiella pneumoniae* (at a dose of 3.0×10⁶ CFU) at a dose of 32 mg/kg through the tail vein at 1 h and 6 h respectively after the infection, the seven-day survival rate of the mice was 60%; after injecting the compound of formula (I-1) to the mice at a dose of 16 mg/kg through the tail vein at 1 h and 6 h respectively after the infection, the seven-day survival rate of the mice was 20%.

Avibactam itself has no *in vivo* antibacterial effect on this test strain, but its combined administration with the compound of formula (I-1) can greatly enhance the *in vivo* antibacterial activity of the latter. After administering the compounds of formula (I-1) at doses of 32 mg/kg and 16 mg/kg each in combination with equal, double and triple amounts of avibactam, respectively, the seven-day survival rate of the mice reached 100%. This indicates that the combination of the compound of formula (I-1) and avibactam can exhibit significant synergistic antibacterial effects *in vivo.*

While preferred embodiments of the present invention have been shown and described herein, it will be obvious to those skilled in the art that such embodiments are provided by way of example only. Numerous variations, changes, and substitutions will now occur to those skilled in the art without departing from the present invention. It should be understood that various alternatives to the embodiments of the present invention described herein may be employed in practicing the present invention. It is intended that the following claims define the scope of the present invention and that methods and structures within the scope of these claims and their equivalents be covered thereby.

## Claims

1. A pharmaceutical composition comprising
(a) a compound of formula (I), or a stereoisomer, a solvate or a pharmaceutically acceptable salt or ester thereof, 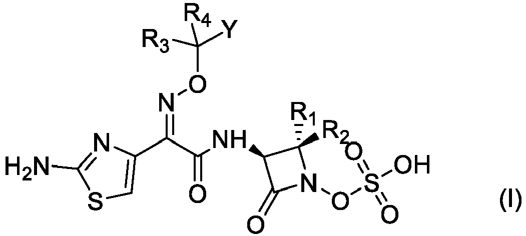 wherein
R₁ and R₂ each independently represent hydrogen, alkyl, aryl, alkoxy, aryloxy, arylalkyl, alkylcarbonylalkyl, alkylacyloxyalkyl or alkoxyacyloxyalkyl, heterocyclyl or heteroaryl, or R₁ and R₂ together form cycloalkyl, wherein all the aforementioned groups are optionally substituted;
R₃ and R₄ each independently represent hydrogen, alkyl, aryl, alkoxy, aryloxy, arylalkyl, alkylcarbonylalkyl, alkylacyloxyalkyl or alkoxyacyloxyalkyl, heterocyclyl or heteroaryl, or R₃ and R₄ together form cycloalkyl, wherein all the aforementioned groups are optionally substituted; and
Y represents optionally substituted alkenyl or alkynyl, or represents carboxyl or ester; and
(b) a lactamase inhibitor or an efflux pump inhibitor.

2. The pharmaceutical composition according to claim 1, wherein in the compound of formula (I),
R₁ and R₂ each independently represent hydrogen, or optionally substituted methyl, ethyl, n-propyl, isopropyl, n-butyl, iso-butyl, sec-butyl or tert-butyl; or R₁ and R₂ together form (C₃-C₆)cycloalkyl;
R₃ and R₄ each independently represent hydrogen, optionally substituted methyl, ethyl, n-propyl, isopropyl, n-butyl, iso-butyl, sec-butyl or tert-butyl, or optionally substituted (C₆-C₁₂)-aryl; or R₃ and R₄ together form (C₃-C₆)cycloalkyl; and
Y represents vinyl, propenyl, isopropenyl, allyl, n-butenyl, isobutenyl, 2-methylpropenyl, ethynyl, propynyl, isopropynyl, propargyl , n-butynyl, isobutynyl, 2-methylpropynyl, carboxyl or -COOR₅, wherein R₅ is (C₁-C₁₂)-alkyl, examples of which are methyl, ethyl, n-propyl, isopropyl, n-butyl, isobutyl, sec-butyl, tert-butyl, n-pentyl, isopentyl, neopentyl, tert-pentyl, 1-methylbutyl, 2-methylbutyl, 1-ethylpropyl, 1,2-dimethylpropyl or hexyl.

3. The pharmaceutical composition according to claim 1 or 2, wherein in the compound of formula (I),
R₁ and R₂ each independently represent hydrogen, or optionally substituted methyl or ethyl;
R₃ and R₄ each independently represent hydrogen, or optionally substituted methyl, ethyl, n-propyl or isopropyl; or R₃ and R₄ together form (C₃-C₆)cycloalkyl; and
Y represents vinyl, propenyl, ethynyl, propynyl, carboxyl or -COOR₅, wherein R₅ is methyl, ethyl, n-propyl or isopropyl.

4. The pharmaceutical composition according to any one of the preceding claims, wherein the compound of formula (I) is selected from

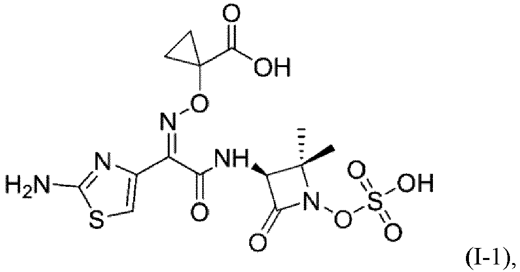

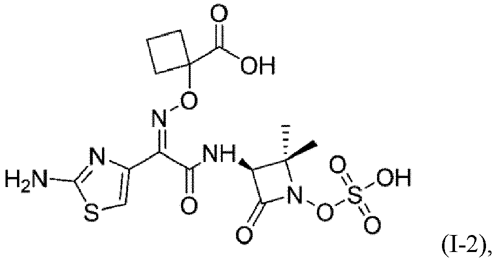

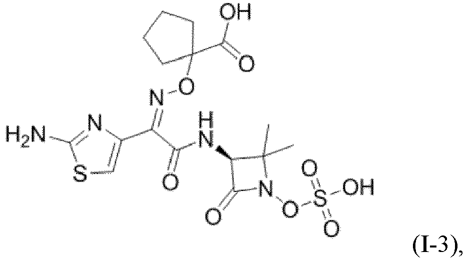

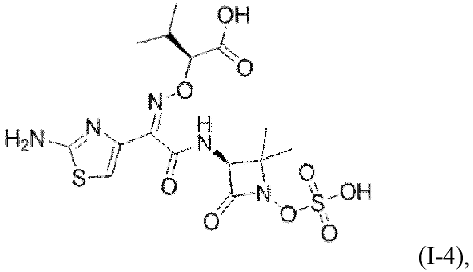

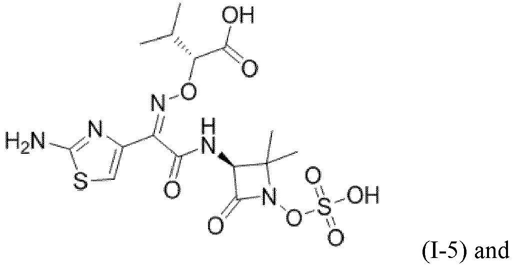

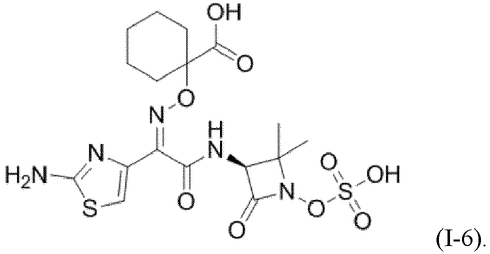

5. The pharmaceutical composition according to any one of the preceding claims, wherein the lactamase inhibitor is selected from the group consisting of avibactam, vaborbactam and relebactam

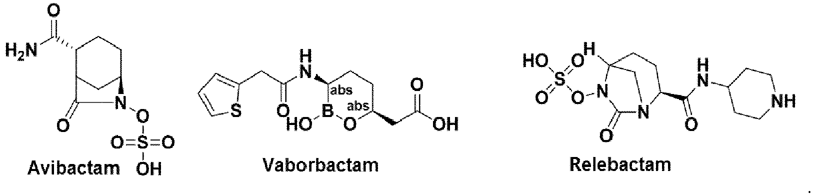

6. The pharmaceutical composition according to any one of the preceding claims, wherein the efflux pump inhibitor is selected from the group consisting of berberine, conessine and PaβN:

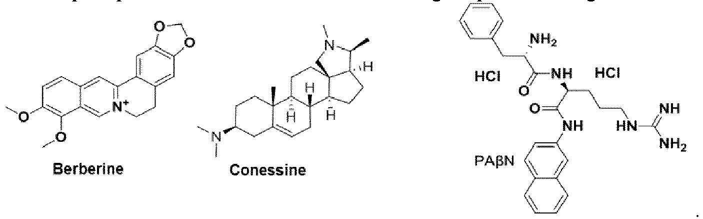

7. The pharmaceutical composition according to any one of the preceding claims, wherein component (a) and component (b) are present in a weight ratio of 1:0.3 to 1:10, preferably 1:0.5 to 1:7, more preferably 1:0.8 to 1:6, most preferably 1: 1 to 1:5.

8. The pharmaceutical composition according to any one of the preceding claims, further comprising a pharmaceutically acceptable carrier, an excipient and/or other adjuvants.

9. Use of the pharmaceutical composition according to any one of the preceding claims as an antimicrobial agent.

10. Use according to claim 9, wherein said microorganism is a bacterium, especially a Gram-negative bacterium, such as *Escherichia coli, Klebsiella pneumoniae, Pseudomonas aeruginosa, Acinetobacter baumannii, Enterobacter cloacae, Enterobacter aerogenes, Salmonella typhi, Serratia marcescens, Citrobacter freundii, Providencia rettgeri, Proteus vulgaris, Proteus mirabilis, Pseudomonas maltophilia* and *Shigella flexneri.*

11. Use according to claim 9 or 10, wherein said microorganism is *Klebsiella pneumoniae, Escherichia coli, Enterobacter cloacae, Acinetobacter baumannii* or *Pseudomonas aeruginosa.*

12. Use according to any one of claims 9 to 11, wherein component (b) is a diazabicyclooctanone β-lactamase inhibitor selected from the group consisting of avibactam, vaborbactam and relebactam 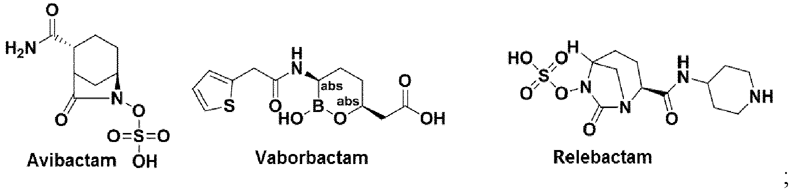 and
said microorganism is selected from *Klebsiella pneumoniae, Escherichia coli* and *Enterobacter cloacae.*

13. Use according to any one of claims 9 to 12, wherein component (b) is an efflux pump inhibitor selected from the group consisting of berberine, conessine and PaN: 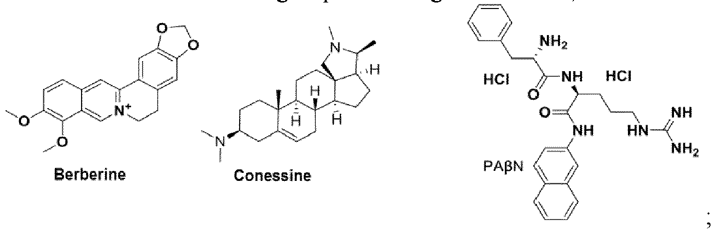 and
said microorganism is selected from *Acinetobacter baumannii* or *Pseudomonas aeruginosa.*

14. Use of the pharmaceutical composition according to any one of claims 1-8 in the manufacture of a medicament for treating infectious diseases.
